# EUROPEAN PATENT APPLICATION

(11) **EP 4 835 477 A1**
(43) Date of publication of application: **04.11.2026**
(21) Application number: 24910668.3
(22) Date of filing: 26.12.2024
(51) Int. Cl.: G01N 33/66, G01N 27/26

(54) **TEST PAPER FOR DETECTING BLOOD GLUCOSE AND PREPARATION METHOD THEREFOR**

(30) Priority: 29.12.2023 CN 202311866845
(71) Applicant: Jiangsu Yuwell POCTech Biotechnology Co., Ltd., Zhenjiang, Jiangsu 212300 (CN)
(72) Inventor: WEI, Liang, Zhenjiang, Jiangsu 212300 (CN); WANG, Caiping, Zhenjiang, Jiangsu 212300 (CN); WANG, Jialin, Zhenjiang, Jiangsu 212300 (CN)
(74) Representative: Lorenz Seidler Gossel Part. mbB
(86) International application number: PCT/CN2024/137907
(87) International publication number: WO 2025/139737

(57) **Abstract**

A blood glucose test strip is provided, including substrate layer (10), an electrode layer, a reagent layer, and a covering layer which are sequentially arranged. The reagent layer includes: 2 to 3 parts of flavin adenine dinucleotide-glucose dehydrogenase (FAD-GDH), 3 to 5 parts of a high polymer, 2 to 3 parts of an electronic mediator, 0.2 to 1 part of a surfactant, and 1 to 2 parts of a buffer solution, where the high polymer includes a first polymer having a degree of polymerization of 200 to 400 and a second polymer having a degree of polymerization of 200 to 300. A preparation method for the blood glucose test strip includes: sputtering a metal layer to the substrate layer (10); etching the metal layer to obtain the electrode layer; and after dropwise adding a reaction liquid to a reaction region of the electrode layer, drying the reaction liquid to obtain the reagent layer, and pasting the covering layer on a top of the electrode layer. Problems of instability and low accuracy due to the "coffee-ring" effect are avoided.

## Description

This application claims priority to Chinese Patent Application No. "202311866845.4", filed with the China National Intellectual Property Administration on December 29, 2023 and entitled "BLOOD GLUCOSE TEST STRIP AND PREPARATION METHOD THEREFOR", which is incorporated herein by reference in its entirety.

### TECHNICAL FIELD

This application relates to a blood glucose test strip and a preparation method therefor, and belongs to the technical field of blood glucose detection.

### BACKGROUND

With the development of blood glucose testing technology, a patient has a growing awareness of self-testing and monitoring blood glucose. The patient can usually test the blood glucose, know blood glucose control of the patient, and provide a reference for adjusting diet and treatment solutions.

At present, on the market, most blood glucose agents are made of a screen-printed carbon electrode material and a metal-coated electrode material. Compared with the screen-printed carbon electrode material, the metal-coated electrode material has beneficial effects that the material is relatively soft and easily processed, a surface of an electrode is hydrophilic, the resistance consistency is good, for example, the sheet resistance accuracy may reach < 1%, and the material is an inert metal material which has high electrical conductivity and is not easily affected by environmental temperature and humidity. However, as a basic material of a blood glucose test strip or other rapid test strips, the metal-coated electrode material has a relatively outstanding problem: since a metal electrode is produced by using a coating process, the surface of the electrode is very uniform and does not have a height difference, and an enzyme liquid is easily diffused irregularly on the surface of the electrode. After curing in a drying channel, a significant "coffee ring" is easily formed. That is, in a process of drying liquid droplets, an evaporation velocity at edges of the liquid droplets is greater than an evaporation velocity at centers of the liquid droplets. Therefore, a supplementary water flow from the center to the edge is generated inside the liquid droplet. Under the driving of the supplementary water flow, suspended particulate matters in the liquid droplet are carried to the edge of the liquid droplet and are deposited. After the liquid droplet is completely dried, the particulate matters can be deposited to form a ring along a three-phase contact line. Consequently, a central enzyme layer is thinned. During testing of high-concentration blood glucose, disadvantages such as a low current and poor current consistency are caused. This affects the stability and accuracy of the test strip.

### SUMMARY

To solve the above problems, the present application provides a blood glucose test strip and a preparation method for the blood glucose test strip, thus solving a problem that the test strip is unstable and has low accuracy due to a "coffee ring" effect when a metal-coated electrode material is used as a material of the blood glucose test strip.

According to one aspect of the present application, a blood glucose test strip is provided. The test strip includes a substrate layer, an electrode layer, a reagent layer, and a covering layer that are sequentially arranged. The reagent layer includes: 2 to 3 parts of flavin adenine dinucleotide-glucose dehydrogenase (FAD-GDH), 3 to 5 parts of a high polymer, 2 to 3 parts of an electronic mediator, 0.2 to 1 part of a surfactant, and 1 to 2 parts of a buffer solution.

The high polymer includes a first polymer having a degree of polymerization of 200 to 400 and a second polymer having a degree of polymerization of 200 to 300.

By selecting the polymers having the degrees of polymerization, brittleness after curing due to an excessively high degree of polymerization can be avoided, thereby avoiding cracking and detachment phenomena after an enzyme layer is cured. In addition, a phenomenon that an effective component cannot be fixedly fixed to an electrode due to an excessively low degree of polymerization.

Optionally, a weight ratio of the first polymer to the second polymer is 1: 2 to 4.

Specifically, the first polymer and the second polymer are independently selected from at least one of sodium carboxymethyl cellulose, hydroxyethyl cellulose, hydroxypropyl cellulose, and polyvinylpyrrolidone.

Optionally, the first polymer is sodium carboxymethyl cellulose having a degree of polymerization of 300 to 400; and the second polymer is hydroxypropyl cellulose having a degree of polymerization of 200 to 300. By selecting the two polymers with different degrees of polymerization to control a crystallization velocity, a polymer with a high crystallization velocity is preferentially crystalized to slow down or hinder liquid from flowing towards the edge, thus mitigating the "coffee-ring effect".

Optionally, the electronic mediator is at least one of a ruthenium compound, phenazine methosulfate, and thionine. Preferably, the electronic mediator includes the phenazine methosulfate and the thionine in a weight ratio of 1:0.2 to 0.5; and/or
the surfactant includes 0.2 to 0.5 parts of cetyltrimethylammonium bromide and 0.2 to 0.5 parts of Trition 100; and/or
the reagent layer further includes 0.5 to 1 part of trehalose and 0.3 to 0.5 parts of potassium chloride.

Since an electroactive center of dehydrogenase is deeply buried, common electron mediators cannot transfer electrons generated in enzymatic reactions. Therefore, the phenazine methosulfate (PMS) is used as a hydrogen carrier to transfer electrons from the active center of the dehydrogenase. The thionine has good molecular structural stability and contains a plurality of electron donors and acceptors, so that highly efficient electron transferring can be implemented. Through the synergistic effect of the PMS and the thionine, electrons produced by a reaction between glucose and enzyme can be transferred to the surface of the electrode, thus implementing quantitative monitoring of blood glucose based on a quantity of transferred electrons.

A spreading capability of the reaction liquid is adjusted by adding the surfactant, so as to form a uniform enzyme layer. The trehalose is used as an enzyme protective agent, to maintain the activity and stability of the enzyme. The potassium chloride provides a suitable micro environment for the enzyme, to ensure that the enzyme exerts a highest activity. The components cooperate with each other, and jointly ensure that the test strip has relatively high test sensitivity.

Optionally, the reagent layer includes: 2 parts of the FAD-GDH, 1 part of the sodium carboxymethyl cellulose, 3 parts of the hydroxypropyl cellulose, 1.4 parts of the phenazine methosulfate, 0.6 parts of the thionine, 0.5 parts of the cetyltrimethylammonium bromide, 0.2 parts of the Trition 100, 1 part of the trehalose, 1 part of a phosphate buffer system, and 90 parts of water.

Optionally, the electrode layer includes a pair of reaction electrode, a pair of hematocrit test electrode, and a sample injection detection electrode.

Specifically, a width between adjacent electrodes in the electrode layer is 60 to 100 µm. A diffusion velocity of the reaction liquid is reduced by controlling a width between adjacent electrodes.

According to another aspect of the present application, a method for preparing the blood glucose test strip described above is provided, including the following steps: sputtering a metal layer to the substrate layer; etching the metal layer to obtain an electrode layer; after dropwise adding a reaction liquid to a reaction region of the electrode layer, drying the reaction liquid to obtain the reagent layer; and pasting the covering layer on a top of the electrode layer.

A drying temperature of the drying process gradually decreases.

Optionally, the drying process includes a high-temperature stage, a medium-temperature stage, and a low-temperature stage.

Specifically, a temperature in the high-temperature stage is 60 to 75°C, a temperature in the medium-temperature stage is 40 to 50°C, and a temperature in the low-temperature stage is 30 to 35°C.

Preferably, the temperature in the high-temperature stage is 65°C, the temperature in the medium-temperature stage is 50°C, and the temperature in the low-temperature stage is 30°C.

An early stage of the drying stage is the high-temperature stage, so as to accelerate the volatilization of a solvent and promote rapid crystallization of a crystallized material, so as to prevent the liquid from flowing to an edge, thereby ensuring that the reagent layer with a uniform thickness is finally formed. Later, by setting the medium-temperature stage and the low-temperature stage, the drying temperature gradually decreases to reduce the curing rate and mitigate the coffee-ring effect.

Optionally, total duration of the drying process is 20 to 30 min.

Preferably, duration of the high-temperature stage is 3 to 5 min, duration of the medium-temperature stage is 10 to 15 min, and duration of the low-temperature stage is 5 to 10 min.

Optionally, after the metal layer is sputtered to the substrate layer, a surface of the metal layer is treated by using a plasma air source to reduce a contact angle of the surface of the metal layer to 40° to 70°, preferably 45° to 55°. The contact angle of the surface of the metal layer is reduced to enhance the hydrophilicity of the surface of the metal layer, thereby reducing a thickness of a liquid droplet, improving the spreading uniformity of the liquid droplet, and increasing an initial crystallization velocity of the crystallized material.

Beneficial effects of the present application include, but are not limited to:
1. According to the blood glucose test strip provided in the present application, the reagent layer includes the FAD-GDH, the high polymer, the electronic mediator, the surfactant, and the buffer solution. The FAD-GDH can quickly and accurately catalyze the oxidation reaction of glucose, thereby improving the sensitivity of the test strip, and the sensitivity, stability, and accuracy of the test strip can be improved. Different crystallization velocities of the high polymer slow down or hinder the flowing of the liquid to the edge, and mitigate a phenomenon of irregular diffusion of an enzyme solution. The electronic mediator helps to promote the transferring of the electrons produced in the oxidation reaction of the glucose. The surfactant can improve the wettability between the reagent layer and blood and is conductive to quick diffusion of the blood and full contact of the reagent layer, thus enhancing the accuracy of test. By the above settings, the performance of the blood glucose test strip can be enhanced.
2. According to the preparation method for the blood glucose test strip provided in the present application, the method is simple and easily implemented, including the steps such as sputtering the metal layer, obtaining the electrode layer by etching, dropwise adding a reaction solution, performing drying, and pasting the covering layer. This can efficiently prepare the blood glucose test strip.
3. According to the preparation method for the blood glucose test strip provided in the present application, in the drying process, a gradual curing and drying temperature process is used. It is conducive to maintaining the stability and activity of the components in the test strip, and slowing down a phenomenon that the supplementary water flow from the center to the edge can drive the suspended particulate matters in the liquid droplet to move to the edge of the liquid droplet and be deposited. By treating the surface of the metal layer with the plasma air source, the contact angle is reduced. This is conductive to forming a uniform and stable cured enzyme layer and enhancing the surface consistency, so that the blood more is easily absorbed, and the test accuracy is enhanced.

### BRIEF DESCRIPTION OF THE DRAWINGS

The accompanying drawings described herein are used to provide a further understanding of the present application, and form part of the present application. Exemplary embodiments of the present application and descriptions thereof are used to explain the present application, and do not constitute any inappropriate limitation to the present application. In the accompanying drawings:
FIG. 1 is a schematic diagram of a layered structure of a blood glucose test strip according to the embodiments of the present application;
FIG. 2 is a diagram of a blood glucose test strip under a microscope according to Embodiment 1 of the present application;
FIG. 3 is a graph of a height distribution curve of an enzyme membrane of a blood glucose test reagent according to an embodiment of the present application;
FIG. 4 is a diagram of a blood glucose test strip under a microscope according to Comparative example 1 of the present application;
FIG. 5 is a diagram of a blood glucose test strip under a microscope according to Comparative example 2 of the present application;
FIG. 6 is a schematic diagram of a height distribution curve of an enzyme membrane of a blood glucose test reagent according to Comparative example 2 of the present application;
FIG. 7 is a diagram of a height distribution curve of an enzyme membrane of a blood glucose test reagent according to Comparative example 3 of the present application; and
FIG. 8 is a diagram of a linear relationship between a blood glucose concentration and a current of a test strip prepared in Embodiment 1 of the present application.

### List of parts and reference numerals:

1: first reaction electrode; 2: second reaction electrode; 3: first hematocrit test electrode; 4: second hematocrit test electrode; 5: a sample injection detection electrode; 6: vent hole; 7: integrated hydrophilic spacer layer; 8: die-cut double-sided adhesive layer; and 9: hydrophilic film layer.

### DETAILED DESCRIPTION

To more clearly describe the overall idea of the present application, the following detailed description is exemplarily provided with reference to the accompanying drawings of this specification.

The following further describes the present application with reference to specific embodiments. It is pointed out that the following embodiments cannot be construed as a limitation to the protection scope of the present application, and some simple replacements or adjustments made by a person of ordinary skill in the art according to content of the present application shall fall within the protection scope of the present application.

Raw materials in the embodiments of the present application are all purchased commercially unless otherwise specified.

To understand the above objectives, features, and advantages of the present application more clearly, the following further describes the present application in detail with reference to the accompanying drawings and specific implementations. It should be noted that the embodiments of the present application and the features in the embodiments may be mutually combined without conflicts.

Many specific details have been elaborated in the following description to facilitate a full understanding of the present application, but the present application can also be implemented in other ways different from those described here. Therefore, the protection scope of the present application will not be limited by the following specific embodiments disclosed.

In addition, in the descriptions of the present application, it should be understood that orientations or positional relationships indicated by "center", "upper", "lower", "front", "rear", "left", "right", "vertical", "horizontal", "top", "bottom", "inner", "outer", "axial", "radial", "circumferential", and the like are orientations or positional relationships as shown in the drawings, and are only for the purpose of facilitating and simplifying the descriptions of the present application instead of indicating or implying that devices or elements indicated must have particular orientations, and be constructed and operated in the particular orientations, so that these terms are not construed as limiting the present application.

In addition, the terms "first" and "second" are used for descriptive purposes only and are not to be construed as indicating or implying relative importance or to implicitly indicate the number of technical features indicated. Thus, features defined as "first" and "second" explicitly or implicitly include one or more of the features. In the descriptions of the present application, "a plurality of" means two or more, unless otherwise definitely and specifically limited.

In the present application, unless otherwise expressly specified and limited, the terms "mount", "connect", "connection", "fix", and the like should be understood in a broad sense, such as, a fixed connection, a detachable connection, an integrated connection, a mechanical connection, an electrical connection, communication, a direct connection, an indirect connection through an intermediate medium, an internal communication of two elements, or interaction between two elements. Those of ordinary skill in the art can understand the specific meanings of the above terms in the present application according to specific situations.

In the present application, unless otherwise explicitly stipulated and restricted, that a first feature is "on" or "below" a second feature may include that the first and second features are in direct contact, or may include that the first and second features are in indirect contact using an intermediate. In the description of this specification, the description referring to the terms "one embodiment", "some embodiments", "an example", "specific examples", "some examples", or the like means that specific features, structures, materials or characteristics described in connection with the embodiments or examples are included in at least one embodiment or example of the present application. In this specification, the schematic representations of the above terms are not necessarily intended to refer to the same embodiment or example. Furthermore, the specific features, structures, materials or characteristics described may be combined in any suitable manner in any one or more embodiments or examples.

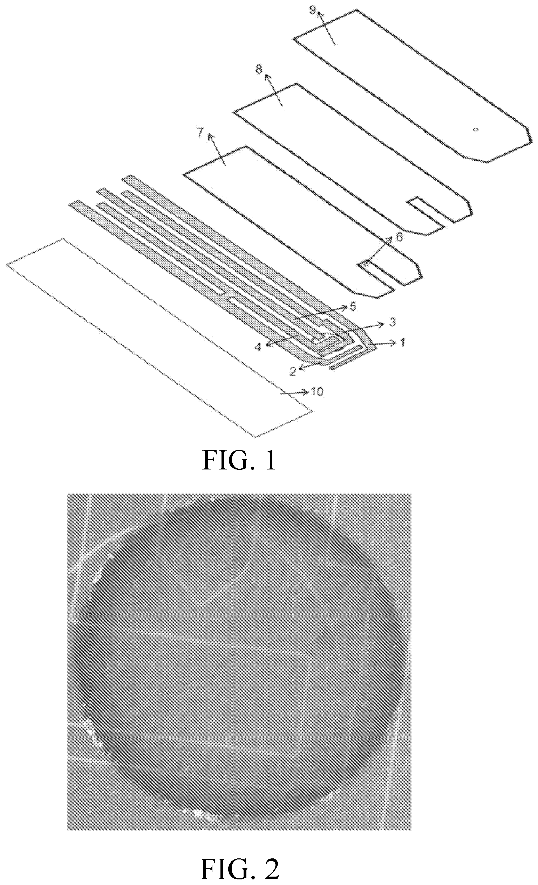

In an exemplary embodiment of the present application, FIG. 1 shows a structure of a blood glucose test strip. The test strip includes, sequentially from bottom to top, a substrate layer 10, an electrode layer, a reagent layer, and a covering layer. The covering layer includes a die-cut double-sided adhesive layer 8 and a hydrophilic film layer 9. The two layers form an integrated hydrophile layer 7 and are arranged above the reagent layer. A vent hole 6 is formed in one side of the hydrophilic film layer 9 close to the reagent layer. The electrode layer includes a pair of reaction electrodes (that is, a first reaction electrode 1 and a second reaction electrode 2), a pair of hematocrit test electrodes (that is, a first hematocrit test electrode 3 and a second hematocrit test electrode 4), and a sample injection detection electrode 5. The reagent layer covers the reaction electrodes and the sample injection detection electrode. A siphon reaction region is formed by the reaction electrodes, the hematocrit test electrodes, and the sample injection detection electrode. A siphon cell is formed by a hollow portion of the die-cut double-sided adhesive layer, the hydrophilic film layer, and the vent hole in the hydrophilic film layer.

In an exemplary embodiment of the present application, a preparation method for a blood glucose test strip includes the following steps:
(1) sputtering a metal layer to a substrate layer, treating a surface of the metal layer by using a plasma air source to reduce a contact angle of the surface of the metal layer to 40° to 70°;
(2) etching the metal layer to obtain an electrode layer, where the electrode layer includes a pair of reaction electrodes, a pair of hematocrit test electrodes, and a sample injection detection electrode, and a width between adjacent electrodes in the electrode layer is 60 to 100 um;
(3) dropwise adding a reaction liquid to a reaction region of the electrode layer drop-wise, where the reaction liquid includes 2 to 3 parts of FAD-GDH, 3 to 5 parts of a high polymer, 2 to 3 parts of an electronic mediator, 0.2 parts to 1 part of a surfactant, and 1 part to 2 parts of a buffer solution; the high polymer includes a first polymer having a degree of polymerization of 200 to 400 and a second polymer having a degree of polymerization of 200 to 300; a weight ratio of the first polymer to the second polymer is 1: 2 to 4; the electronic mediator includes phenazine methosulfate and thionine in a weight ratio of 1:0.2 to 0.5; the surfactant includes 0.2 to 0.2 to 0.5 parts of cetyltrimethylammonium bromide and 0.2 to 0.5 parts of Trition 100; the reaction liquid further includes 0.5 to 1 part of trehalose and 0.3 to 0.5 parts of potassium chloride;
(4) drying the reaction liquid to obtain a reagent layer, where the drying process includes a high-temperature stage, a medium-temperature stage, and a low-temperature stage; a temperature of the high-temperature stage is 60°C to 75°C, and duration is 3 to 5 min; a temperature of the medium-temperature stage is 40°C to 50°C, and duration is 10 to 15 min; and a temperature of the low-temperature stage is 30°C to 35°C, and duration is 5 to 10 min; and
(5) pasting a covering layer on a top of the electrode layer.

### Embodiment 1

(1) A metal layer was sputtered to a substrate layer. A surface of the metal layer was treated by using a plasma air source to reduce a contact angle of the surface of the metal layer to 55°.
(2) The metal layer was etched to obtain an electrode layer, where the electrode layer included a pair of reaction electrodes, a pair of hematocrit test electrodes, and a sample injection detection electrode, and a width between adjacent electrodes in the electrode layer was 80 um.
   After reaction liquid was dropwise added to a reaction region of the electrode layer, the reaction liquid was dried to obtain a reagent layer, where the reaction liquid included: 2 parts of FAD-GDH, 1 part of sodium carboxymethyl cellulose having a degree of polymerization of 300, 3 parts of hydroxypropyl cellulose having a degree of polymerization of 200, 1.4 parts of phenazine methosulfate, 0.6 parts of thionine, 0.5 parts of cetyltrimethylammonium bromide, 0.2 parts of Trition 100, 1 part of trehalose, 1 part of a phosphate buffer system, and 90 parts of water.
(3) The reaction liquid was dried to obtain the reagent layer, where the drying process included a high-temperature stage, a medium-temperature stage, and a low-temperature stage; a temperature of the high-temperature stage was 65°C, and duration was 4 min; a temperature of the medium-temperature stage was 45°C, and duration was 12 min; and a temperature of the low-temperature stage was 32°C, and duration was 8 min.
(4) A covering layer was pasted on a top of the electrode layer.

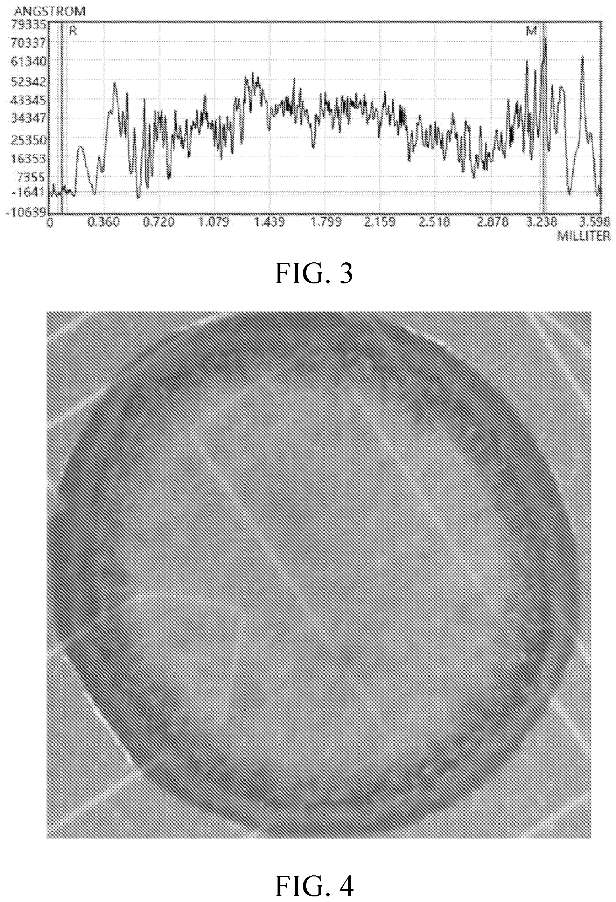

An enzyme membrane prepared in this embodiment was observed by using a 200× magnifier. As shown in FIG. 2, it can be seen that a center of the entire liquid was radiated outwards after drying, with a uniform color, and the entire enzyme membrane was uniform. A height distribution of the enzyme membrane was represented by using a profilometer. As shown in FIG. 3, the overall height distribution of the enzyme membrane was uniform, and an average height was about 5 um.

### Embodiment 2

Embodiment 2 differed from Embodiment 1 in that in step (3), the temperature of the high-temperature stage was 65°C and the duration was 12 min; and the temperature of the low-temperature stage was 32°C and the duration was 12 min. The remaining steps were the same as those in Embodiment 1.

### Embodiment 3

A difference from Embodiment 1 was that in step (3), the temperature of the high-temperature stage was 75°C; the temperature of the medium-temperature stage was 40°C; and the temperature of the low-temperature stage was 30°C. The remaining steps were the same as those in Embodiment 1.

### Embodiment 4

A difference from Embodiment 1 was that in step (1), the surface of the metal layer was treated by not using the plasma air source.

The remaining steps were the same as those in Embodiment 1.

### Embodiment 5

A difference from Embodiment 1 was that in step (2), in the high polymer of the reaction liquid, 2 parts of sodium carboxymethyl cellulose having a degree of polymerization of 300 and 4 parts of hydroxypropyl cellulose having a degree of polymerization of 200 were used to replace the 1 part of sodium carboxymethyl cellulose having the degree of polymerization of 300 and the 3 parts of hydroxypropyl cellulose having the degree of polymerization of 200.

The remaining steps were the same as those in Embodiment 1.

### Embodiment 6

A difference from Embodiment 1 was that in step (2), the electronic mediator in the reaction liquid selected 3 parts of a ruthenium compound.

The remaining steps were the same as those in Embodiment 1.

### Embodiment 7

A difference from Embodiment 1 was that in step (2), the electronic mediator in the reaction liquid selected 2 parts of thionine.

The remaining steps were the same as those in Embodiment 1.

### Comparative example 1

A difference from Embodiment 1 was that in step (2), in the high polymer of the reaction liquid, 1 part of sodium carboxymethyl cellulose having a degree of polymerization of 200 and 3 parts of hydroxypropyl cellulose having a degree of polymerization of 500 were used to replace the 1 part of sodium carboxymethyl cellulose having the degree of polymerization of 300 and the 3 parts of hydroxypropyl cellulose having the degree of polymerization of 200.

The remaining steps were the same as those in Embodiment 1.

An enzyme membrane prepared in this embodiment was observed by using a 200× magnifier. As shown in FIG. 4, it can be seen that the color of the edge of the enzyme membrane and the color of the middle portion of the enzyme membrane were inconsistent, showing a state in which the overall color was not uniformly distributed. This indicated that the distribution of the enzyme membrane was not uniform.

### Comparative example 2

A difference from Embodiment 1 was that in step (2), in the high polymer of the reaction liquid, 4 parts of sodium carboxymethyl cellulose having a degree of polymerization of 300 was used to replace the 1 part of sodium carboxymethyl cellulose having the degree of polymerization of 300 and the 3 parts of hydroxypropyl cellulose having the degree of polymerization of 200.

The remaining steps were the same as those in Embodiment 1.

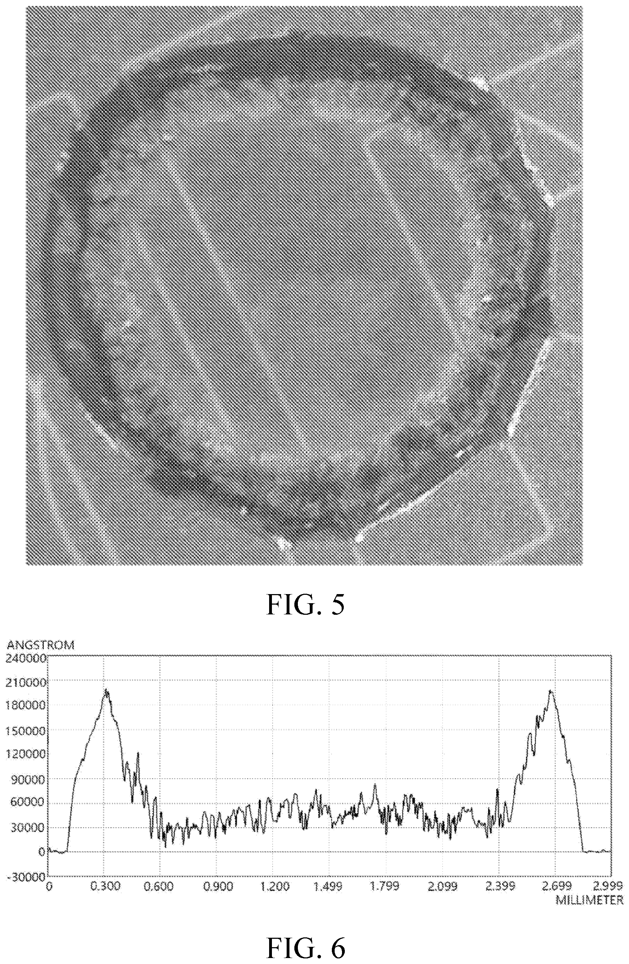

An enzyme membrane prepared in this embodiment was observed by using a 200× magnifier. As shown in FIG. 5, it can be seen that the color of the edge of the enzyme membrane and the color of the middle portion of the enzyme membrane were inconsistent, showing a state in which the overall color was not uniformly distributed. In addition, a large number of crystals were produced at the edge, and the center was hollowed out. This indicated that the distribution of the enzyme membrane was not uniform. A height distribution of the enzyme membrane was represented by using a profilometer. As shown in FIG. 6, it can be seen that the enzyme membrane had a height distribution in which two sides were high and the middle was low. The two sides had a height of about 20 um, and the middle had an average height of about 3 um.

### Comparative example 3

A difference from Embodiment 1 was that in step (3), in the drying process, drying was performed for 24 min at 45°C.

The remaining steps were the same as those in Embodiment 1.

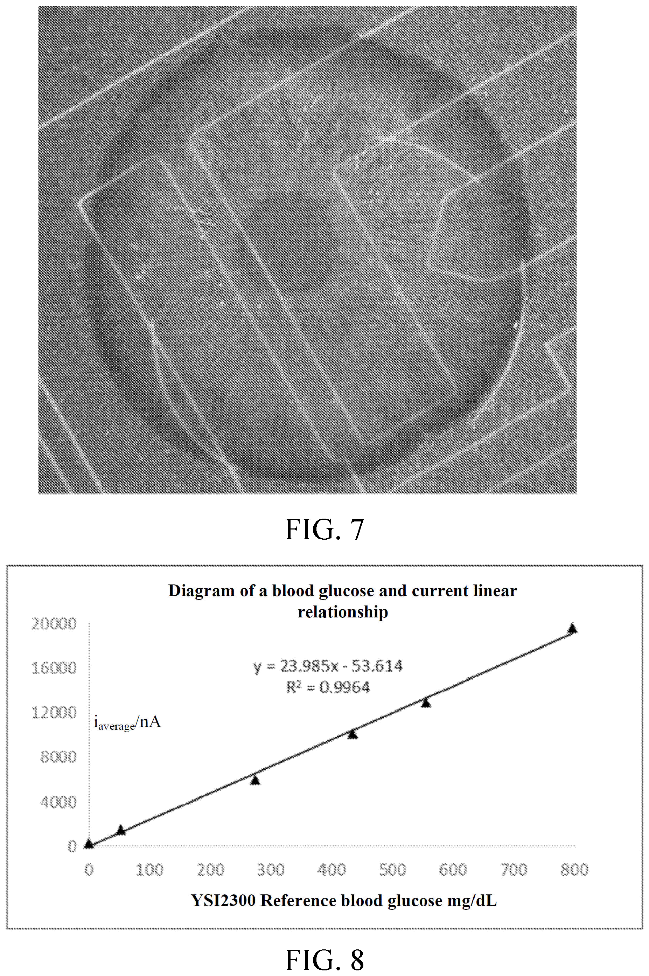

An enzyme membrane prepared in this embodiment was observed by using a 200× magnifier. As shown in FIG. 7, it can be seen that the color of the edge of the enzyme membrane and the color of the middle portion of the enzyme membrane were inconsistent, showing a state in which the overall color was not uniformly distributed, and the center of the enzyme membrane was hollowed out. This indicated that the distribution of the enzyme membrane was not uniform.

### Experiments

The blood glucose test strips prepared in the foregoing embodiments 1 to 7 and the foregoing comparative examples 1 to 3 were connected to a test circuit of a blood glucose meter. Blood samples with different blood glucose concentrations were prepared in a laboratory, to perform a test using the test strips. Sensitivities of the blood glucose test strips for testing blood glucose were tested, to obtain values of R2, intercept/slope, and the coefficient of variation of repeatability CV% of a fitted equation, thus obtaining a statistical table of test data shown in Table 1.

**Table 1**

| Sample | Sensitivity (nA/mg/dL) | R2 | Intercept/slope | Average coefficient of variation CV% |
|---|---|---|---|---|
| Embodiment 1 | 23.985 | 0.9964 | -2.235 | 2.4% |
| Embodiment 2 | 22.985 | 0.9928 | 4.246 | 3.1% |
| Embodiment 3 | 20.384 | 0.99 | 2.296 | 3.8% |
| Embodiment 4 | 23.009 | 0.9879 | 22.01 | 4.7% |
| Embodiment 5 | 22.241 | 0.9945 | 12.73 | 4.0% |
| Embodiment 6 | 23.341 | 0.9887 | 13.98 | 4.5% |
| Embodiment 7 | 22.12 | 0.9908 | 17.91 | 4.3% |
| | | | | |
| Comparative example 1 | 20.925 | 0.9895 | 18.94 | 5.0% |
| Comparative example 2 | 21.077 | 0.9898 | 16.34 | 5.3% |
| Comparative example 3 | 19.403 | 0.9912 | 19.59 | 5.9% |

When R2 is closer to 1, it indicates a better fitting effect. The value of the intercept/slope represents the impact of the background current on a fitting result of blood glucose at a low concentration. A larger value of the intercept/slope represents higher impact. When the intercept/slope is 0, it means that the intercept is 0, represents that a fitted curve passes the origin, and indicates that the background current is 0. The average coefficient of variation represents the consistency of test results of 10 test strips. A smaller coefficient of variation indicates a smaller difference between the test strips. It can be seen from the above table that the blood glucose test strip prepared in the present application has high sensitivity and low background current, and the test results of the blood glucose test strip are highly consistent. In addition, FIG. 8 is a diagram of a linear relationship between a blood glucose concentration and a current of a test strip prepared in Embodiment 1 of the present application. It can be seen from the diagram that the test strip has a relatively good fitting effect, low background current, and higher sensitivity.

The above descriptions are merely embodiments of the present application. The protection scope of the present application is not limited to these specific embodiments, but is determined according to the claims of the present application. Those skilled in the art can make various changes and modifications to the present application. Any modification, equivalent replacement, and improvement made within the spirit and scope of the present application shall fall within the protection scope of the present application.

## Claims

1. A blood glucose test strip, wherein the test strip comprises a substrate layer, an electrode layer, a reagent layer, and a covering layer that are sequentially arranged; the reagent layer comprises: 2 to 3 parts of flavin adenine dinucleotide-glucose dehydrogenase (FAD-GDH), 3 to 5 parts of a high polymer, 2 to 3 parts of an electronic mediator, 0.2 to 1 part of a surfactant, and 1 to 2 parts of a buffer solution; and
the high polymer comprises a first polymer having a degree of polymerization of 200 to 400 and a second polymer having a degree of polymerization of 200 to 300.

2. The blood glucose test strip according to claim 1, wherein a weight ratio of the first polymer to the second polymer is 1: 2 to 4; and
the first polymer and the second polymer are independently selected from at least one of sodium carboxymethyl cellulose, hydroxyethyl cellulose, hydroxypropyl cellulose, and polyvinylpyrrolidone.

3. The blood glucose test strip according to claim 2, wherein the first polymer is sodium carboxymethyl cellulose having a degree of polymerization of 300 to 400; and the second polymer is hydroxypropyl cellulose having a degree of polymerization of 200 to 300.

4. The blood glucose test strip according to claims 1 to 3, wherein the electronic mediator is at least one of a ruthenium compound, phenazine methosulfate, and thionine; preferably, the electronic mediator comprises the phenazine methosulfate and the thionine in a weight ratio of 1:0.2 to 0.5; and/or the surfactant comprises 0.2 to 0.5 parts of cetyltrimethylammonium bromide and 0.2 to 0.5 parts of Trition 100; and/or the reagent layer further comprises 0.5 to 1 part of trehalose and 0.3 to 0.5 parts of potassium chloride.

5. The blood glucose test strip according to claim 4, wherein the reagent layer comprises: 2 parts of the FAD-GDH, 1 part of the sodium carboxymethyl cellulose, 3 parts of the hydroxypropyl cellulose, 1.4 parts of the phenazine methosulfate, 0.6 parts of the thionine, 0.5 parts of the cetyltrimethylammonium bromide, 0.2 parts of the Trition 100, 1 part of the trehalose, 1 part of a phosphate buffer system, and 90 parts of water.

6. The blood glucose test strip according to any one of claims 1 to 3, wherein the electrode layer comprises a pair of reaction electrode, a pair of hematocrit test electrode, and a sample injection detection electrode; and
a width between adjacent electrodes in the electrode layer is 60 to 100 µm.

7. A method for preparing the blood glucose test strip according to any one of claims 1 to 6, comprising the following steps: sputtering a metal layer to the substrate layer; etching the metal layer to obtain an electrode layer; after dropwise adding a reaction liquid to a reaction region of the electrode layer, drying the reaction liquid to obtain the reagent layer; and pasting the covering layer on a top of the electrode layer, wherein a drying temperature of the drying process gradually decreases.

8. The method according to claim 7, wherein the drying process comprises a high-temperature stage, a medium-temperature stage, and a low-temperature stage;
a temperature in the high-temperature stage is 60 to 75°C, a temperature in the medium-temperature stage is 40 to 50°C, and a temperature in the low-temperature stage is 30 to 35°C; and
preferably, the temperature in the high-temperature stage is 65°C, the temperature in the medium-temperature stage is 45°C, and the temperature in the low-temperature stage is 32°C.

9. The method according to claim 8, wherein total duration of the drying process is 20 to 30 min; and
preferably, duration of the high-temperature stage is 3 to 5 min, duration of the medium-temperature stage is 10 to 15 min, and duration of the low-temperature stage is 5 to 10 min.

10. The method according to claim 7, wherein after the metal layer is sputtered to the substrate layer, a surface of the metal layer is treated by using a plasma air source to reduce a contact angle of the surface of the metal layer to 40° to 70°, preferably 45° to 55°.
